# FASCICULE DE BREVET EUROPEEN

(11) **EP 2 731 703 B1**
(45) Date de publication et mention de la délivrance du brevet: **23.08.2017**
(21) Numéro de dépôt: 12744067.5
(22) Date de dépôt: 09.07.2012
(51) Int. Cl.: B01J 8/00, B01J 8/18, B01J 8/38, B01J 8/22, B01J 38/30, B01J 23/92, B01J 27/28, C07C 45/38, C07C 45/52, C07C 46/06, C07C 47/22, C07C 51/235, C07C 51/265, C07C 51/31, C07C 51/377, C07C 57/04, C07C 57/145, C07C 63/16, C07C 63/26, C07C 51/215

(54) **RÉACTEUR A LIT FLUIDISÉ COMPRENANT UNE ZONE DE RÉACTION ET UNE ZONE DE RÉGÉNÉRATION DE CATALYSEUR CONTINUE EN LIT FLUIDISÉ**
FLIESSBETTREAKTOR MIT EINER REAKTIONSZONE UND EINER KONTINUIERLICHEN KATALYSATORREGENERATIONSZONE IN EINEM FLIESSBETTREAKTOR
FLUIDIZED BED REACTOR WITH A REACTION ZONE ET A CONTINUOUS CATALYST FLUIDIZED BED REGENERATION SECTION

(30) Priorité: 12.07.2011 FR 1156312
(43) Date de publication de la demande: 21.05.2014
(73) Titulaire: Arkema France, 92700 Colombes (FR)
(72) Inventeur: DUBOIS, Jean-Luc, F-69390 Millery (FR); PATIENCE, Grégory, Ville Mont-Royal, Québec H3P 1N8 (CA)
(74) Mandataire: Bonnel, Claudine
(86) Numéro de dépôt international: PCT/FR2012/051617
(87) Numéro de publication internationale: WO 2013/007941

(56) Documents cités:
- WO-A1-2009/156655
- FR-A1- 2 920 767
- US-A- 2 477 751
- US-A- 2 698 281
- US-A- 2 872 472
- US-A- 3 057 923
- US-A- 3 669 877
- US-A- 4 623 443
- US-A1- 2003 073 751

## Description

### DOMAINE DE L'INVENTION

La présente invention propose un réacteur catalytique dans lequel la régénération du catalyseur est effectuée en continu, dans l'enceinte du réacteur lui-même. Ce réacteur catalytique est adapté à la mise en oeuvre d'un grand nombre de réactions chimiques.

### ARRIERE-PLAN TECHNIQUE

On connaît l'utilisation de réacteurs catalytiques à lit fluidisé dans les procédés de génie chimique.

Il est également connu de disposer un tube d'aspiration dans l'enceinte d'un réacteur à lit fluidisé, afin de promouvoir la circulation des espèces solides au sein du réacteur (réacteurs de type ICFB).

Il est également connu d'associer le réacteur catalytique à un régénérateur disposé à côté de celui-ci, un flux de catalyseur usagé étant établi du réacteur vers le régénérateur, et un flux de catalyseur régénéré étant établi du régénérateur vers le réacteur. De tels systèmes sont appropriés lorsque le catalyseur se désactive relativement rapidement (typiquement sur une durée de l'ordre de quelques secondes ou minutes) et doit être fréquemment régénéré. Ces systèmes sont notamment utilisés pour la conversion d'hydrocarbures en oléfines oxygénés. Les documents US 2,698,281 A, US 3,669,877 A, US 2007/0203383 et US 2010/0028224 fournissent des exemples de ces systèmes.

L'article de Zhang et al. intitulé Hydrodynamics of a novel biomass autothermal fast pyrolysis reactor: flow pattern and pressure drop, dans Chem. Eng. Technol. 32:27-37 (2009), décrit un réacteur pour la pyrolyse catalytique de biomasse, dans lequel la pyrolyse est effectuée dans une partie centrale, avec un entraînement de biomasse et de catalyseur, tandis que les dépôts sur le catalyseur sont brûlés dans une zone périphérique du réacteur. Toutefois, ce schéma de réacteur n'est pas adapté pour des réactions dans lesquelles le temps de séjour du catalyseur dans la zone de réaction doit être relativement élevé (de l'ordre de quelques heures par exemple) pour une efficacité optimale.

Les réacteurs catalytiques à lit fluidisé de l'état de la technique ne sont pas adaptés pour permettre un contrôle efficace du débit de gaz réactionnel indépendamment du flux de catalyseur destiné à être régénéré, ou pour fournir efficacement de la chaleur au régénérateur, en vue d'optimiser la mise en oeuvre de réactions chimiques en phase gazeuse (US 4,623,443 ; US 3,057,923 ; US 2003/073751 ; US 2,872,472 ; EP 340 852).

Il n'existe pas à l'heure actuelle d'installation suffisamment compacte et permettant un contrôle optimal des conditions opératoires, en particulier pour des réactions dans lesquelles la désactivation du catalyseur présente une durée caractéristique d'un ordre de grandeur supérieur à la minute et inférieur au mois.

### RESUME DE L'INVENTION

L'invention concerne en premier lieu un réacteur catalytique comprenant une enceinte et des moyens de séparation disposés dans l'enceinte, délimitant au moins une zone de réaction et au moins une zone de régénération, la zone de réaction étant alimentée par un dispositif d'alimentation en flux réactionnel et étant connectée en sortie à un premier dispositif de soutirage de gaz, la zone de régénération étant alimentée par un dispositif d'alimentation en flux de régénération et étant connectée en sortie à un deuxième dispositif de soutirage de gaz, le réacteur comportant un lit fluidisé de particules de catalyseur dans la zone de réaction, les moyens de séparation permettant le passage des particules de catalyseur de la zone de réaction vers la zone de régénération, et le réacteur étant adapté à comporter un entraînement de particules de catalyseur dans la zone de régénération permettant leur retour vers la zone de réaction.

Le réacteur selon l'invention est caractérisé en ce que le rapport de la section maximale de lit de catalyseur dans la zone de réaction sur la section maximale de la (ou des) zone(s) de régénération prises collectivement va de 1 à 100, de préférence de 5 à 50.

Selon un mode de réalisation, les moyens de séparation comportent une paroi tubulaire, la zone de réaction est située à l'extérieur de la paroi tubulaire et la zone de régénération est située à l'intérieur de la paroi tubulaire.

Selon un mode de réalisation, le rapport de la section maximale de lit de catalyseur dans la zone de réaction sur la section maximale de la (ou des) zones de régénération prises collectivement est plus particulièrement de 10 à 20.

Selon un mode de réalisation, la zone de régénération comprend une partie supérieure et une partie inférieure, la partie supérieure présentant une section inférieure à celle de la partie inférieure, la partie inférieure étant adaptée à comporter un lit fluidisé de particules catalyseur, et la partie supérieure étant adaptée à entraîner les particules de catalyseur.

Selon un mode de réalisation, le réacteur catalytique comprend une grille inférieure dans l'enceinte adaptée à retenir les particules de catalyseur au-dessus de celle-ci, et un espacement est ménagé entre les moyens de séparation et la grille inférieure, permettant le passage des particules de catalyseur de la zone de réaction vers la zone de régénération.

Selon un mode de réalisation, le réacteur catalytique comprend un chicanage dans la zone de réaction ménageant un espace intermédiaire entre le chicanage et les moyens de séparation.

Selon un mode de réalisation, le réacteur catalytique comprend des moyens d'injection de gaz de contre-pression dans l'espace intermédiaire.

L'invention concerne également un procédé de réaction chimique mis en oeuvre dans un réacteur catalytique tel que décrit ci-dessus, comprenant de manière concomitante :
- l'alimentation par un flux réactionnel d'un lit fluidisé de particules de catalyseur dans une zone de réaction disposée dans une enceinte d'un réacteur,
- le soutirage d'un flux comprenant des produits de réaction en sortie de la zone de réaction,
- le passage, à l'aide de moyens de séparation, de particules de catalyseur de la zone de réaction vers une zone de régénération disposée à l'intérieur de l'enceinte du réacteur,
- l'alimentation de la zone de régénération par un flux de régénération,
- la régénération des particules de catalyseur et l'entraînement pneumatique des particules de catalyseur dans la zone de régénération,
- le soutirage d'un gaz d'échappement en sortie de la zone de régénération, et
- le retour des particules de catalyseur entraînées vers la zone de réaction.

Selon un mode de réalisation, l'alimentation de gaz dans la partie haute du régénérateur (riser) est indépendante de l'alimentation de gaz dans la partie inférieure du régénérateur, permettant ainsi un meilleur contrôle de la perte de charge et donc du débit de solide circulant.

Selon l'invention, ladite réaction chimique est caractérisée en ce que la durée de désactivation du catalyseur est de 1 à 20 fois supérieure à la durée de régénération.

Selon un mode de réalisation, le flux de particules de catalyseur de la zone de réaction vers la zone de régénération est régulé par l'injection d'un gaz de contre-pression.

Selon un mode de réalisation, le flux de régénération comprend de l'oxygène, et de préférence le flux réactionnel comprend de l'oxygène.

Selon un mode de réalisation, le procédé est :
- un procédé de déshydratation du glycérol en acroléine ; ou
- un procédé de déshydratation de l'acide lactique en acide acrylique ; ou
- un procédé de déshydratation de l'acide 3-hydroxypropionique en acide acrylique ; ou
- un procédé de déshydratation de l'acide 3-hydroxyisobutyrique en acide methacrylique ; ou
- un procédé de déshydratation de l'acide 2-hydroxyisobutyrique, encore appelé alpha-hyroxyisobutyrique, en acide methacrylique.

La présente invention permet de surmonter les inconvénients de l'état de la technique. Elle fournit plus particulièrement une installation de réacteur compacte et permettant un contrôle optimal des conditions opératoires. L'invention est particulièrement utile pour des réactions dans lesquelles la désactivation du catalyseur présente une durée caractéristique d'un ordre de grandeur supérieur à la minute et inférieur au mois ; et / ou pour les réactions dans lesquelles la durée de désactivation (définie comme étant la durée de réaction au bout de laquelle le catalyseur a perdu 25 % de son efficacité) est de 1 à 20 fois, de préférence de 1 à 10 fois, supérieure à la durée de régénération (définie comme étant la durée de régénération au bout de laquelle un catalyseur ayant perdu 25 % de son efficacité initiale retrouve jusqu'à environ 100 % de ladite efficacité initiale).

Ceci est accompli grâce à un modèle de réacteur dans lequel la régénération du catalyseur a lieu à l'intérieur même du réacteur, dans une zone dédiée à la régénération, cette zone de régénération opérant en régime d'entraînement des particules de catalyseur.

Selon certains modes de réalisation particuliers, l'invention présente également une ou, de préférence, plusieurs des caractéristiques avantageuses énumérées ci-dessous.
- Dans les lits fluidisés usuels comportant des moyens de circulation interne tels qu'un tube d'aspiration, la perte de charge dans le tube d'aspiration est contrôlée par le débit de gaz. Aussi, toute augmentation du débit de gaz entraîne une augmentation du flux de catalyseur et donc une réduction du temps de séjour du catalyseur. En raison de cette interaction entre débit de gaz et flux de catalyseur, la conception de ces réacteurs est particulièrement complexe. L'invention, en revanche, prévoit, selon un mode de réalisation, l'injection d'un gaz de contre-pression à la jonction entre la zone de réaction et la zone de régénération. Cela permet de contrôler le flux de catalyseur de manière indépendante du débit de gaz, et donc de maintenir un temps de séjour du catalyseur élevé. En d'autres termes, l'invention permet un meilleur contrôle de l'hydrodynamique, notamment dans la zone de régénération, ce qui simplifie la conception du réacteur.
- L'invention permet de séparer les gaz de la zone de réaction et de la zone de régénération, et ainsi en particulier d'éviter les mélanges d'oxygène et d'hydrocarbures pouvant présenter des risques d'explosion, de combustion, d'oxydation partielle ou complète.
- L'injection de gaz de contre-pression permet de limiter le passage vers la zone de régénération de gaz circulant dans la zone de réaction, et donc de limiter les pertes de réactifs ou de produits de réaction.

### BREVE DESCRIPTION DES FIGURES

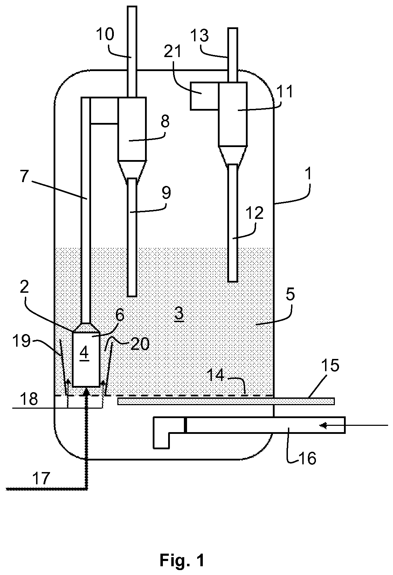

La **figure 1** représente de manière schématique un mode de réalisation d'un réacteur selon l'invention, en coupe.

### DESCRIPTION DE MODES DE REALISATION DE L'INVENTION

L'invention est maintenant décrite plus en détail et de façon non limitative dans la description qui suit.

### Réacteur

En faisant référence à la **figure 1**, le réacteur catalytique selon l'invention comprend une enceinte 1. L'enceinte 1 peut avoir une forme globalement cylindrique à section circulaire, ou cylindrique à section carrée ou rectangulaire ou plus généralement polygonale. De préférence, l'axe principal de l'enceinte (axe du cylindre) est disposé selon la direction verticale, ainsi que cela est illustré. De préférence, le réacteur fonctionne en flux ascendant, c'est-à-dire que le réacteur est alimenté en réactifs en pied et que les produits de la réaction sont prélevés en tête du réacteur.

Selon certains modes de réalisations (non illustrés), l'enceinte 1 peut comporter une forme globalement tubulaire selon l'axe vertical, avec une section variable. Par exemple, une partie supérieure du réacteur peut avoir une section supérieure à une partie inférieure du réacteur. Dans ce cas, la partie supérieure constitue une zone de dégagement du réacteur, ce qui permet de limiter l'entraînement de particules solides dans la partie supérieure du réacteur.

A l'intérieur de l'enceinte 1, des moyens de séparation 2 délimitent une zone de réaction 3 et une zone de régénération 4. Les moyens de séparation 2 comportent en général une paroi ou une pluralité de parois de séparation. Dans le mode de réalisation illustré, les moyens de séparation 2 sont constitués par une paroi de séparation tubulaire, à section circulaire. Alternativement, un ensemble de parois de séparation formant un tube à section rectangulaire ou carrée ou plus généralement polygonale peuvent être utilisés. De préférence, la zone de réaction 3 est située à l'extérieur de la ou les parois tubulaires, et la zone de régénération 4 est située à l'intérieur.

Alternativement, on peut utiliser une ou plusieurs parois de séparation formant un tube conjointement avec une partie de l'enceinte 1. Dans ce cas, la zone de régénération est délimitée à la fois par une partie de l'enceinte 1 et par les parois de séparation.

Dans le mode de réalisation illustré, la zone de régénération 4 est disposée de façon excentrée par rapport à l'axe principal de l'enceinte 1. Pour des raisons de symétrie de l'écoulement, il est toutefois préférable de disposer la zone de régénération 4, lorsqu'elle est unique, dans une position centrale dans l'enceinte 1.

Il est également possible de prévoir une pluralité de zones de régénération 4, chacune étant pourvue de moyens de séparation 2 distincts, dans l'enceinte 1. Dans ce cas, une disposition symétrique ou régulière des différentes zones de régénération 4 par rapport à l'axe principal de l'enceinte 1 est préférée. Une pluralité de zones de régénération 4 permet de faciliter l'extrapolation entre différentes tailles d'unités, en conservant les zones de régénération 4 de taille identique.

Un catalyseur sous forme de particules solides est disposé dans l'enceinte 1. Les moyens de séparation 2 sont prévus de telle sorte qu'une communication existe entre la zone de réaction 3 et la zone de régénération 4 pour la circulation du catalyseur depuis la zone de réaction 3 vers la zone de régénération 4.

Dans le mode de réalisation illustré, le catalyseur est présent dans la zone de réaction 3 sous la forme d'un lit 5 fluidisé au-dessus d'une grille inférieure 14 (dont la taille d'orifices est adaptée à retenir les particules de catalyseur). Un dispositif d'alimentation en flux réactionnel 15, 16 alimente en fluide le lit de catalyseur 5. Il comprend au moins des moyens d'injection de gaz de fluidisation 16 sous la grille inférieure 14. Le gaz de fluidisation est un gaz dont le débit est adapté de telle sorte que le lit de catalyseur 5 soit sous forme fluidisée.

Dans le mode de réalisation illustré, les moyens d'injection de gaz de fluidisation 16 sont orientés vers le bas, de sorte que le gaz de fluidisation est injecté vers le bas de l'enceinte 1 où il est dévié vers le haut, et traverse la grille inférieure 14 puis le lit de catalyseur 5. Cela permet une alimentation plus homogène en gaz de fluidisation. Alternativement, on peut également prévoir que les moyens d'injection de gaz de fluidisation 16 soient directement orientés vers le haut, c'est-à-dire vers le lit de catalyseur 5.

Le dispositif d'alimentation en flux réactionnel 15, 16 peut également comprendre en outre des moyens d'alimentation complémentaires 15 comme cela est illustré. Les moyens d'alimentation complémentaires 15 peuvent être disposés sous la grille inférieure 14, à la verticale de la zone de réaction 3, comme cela est illustré. La grille inférieure 14 peut alors faire office de grille de distribution.

Alternativement, on peut prévoir que les moyens d'alimentation complémentaires 15 soient disposés au-dessus de la grille inférieure 14, c'est-à-dire débouchent directement dans le lit de catalyseur 5, et ce à une seule cote verticale ou de manière étagée à plusieurs niveaux dans le lit de catalyseur 5.

Il est également possible d'effectuer une alimentation étagée à la fois au-dessus et en dessous de la grille inférieure 14.

Les moyens d'alimentation complémentaires 15 peuvent comprendre un ensemble de conduites d'injection munies d'orifices, disposées par exemple en cercles concentriques, ou bien en réseau de lignes parallèles.

Par « flux réactionnel » on entend l'ensemble des composés alimentant la zone de réaction 3. Ce flux réactionnel comprend les réactifs, éventuellement des composés inertes et éventuellement une ou des substances de régénération du catalyseur, par exemple un composé oxydant (et notamment de l'oxygène) afin de prolonger la durée de désactivation du catalyseur. Le flux réactionnel peut être purement gazeux ou comprendre du gaz et du liquide. Dans ce dernier cas, le liquide est vaporisé *in situ* dans l'enceinte 1.

Le flux réactionnel est introduit soit en totalité par les moyens d'injection de gaz de fluidisation 16, soit en partie par ceux-ci et en partie par les moyens d'alimentation complémentaires 15 lorsqu'ils sont présents.

Par exemple, si l'on injecte de l'oxygène en tant que partie du flux réactionnel, il peut être approprié de l'introduire de manière séparée des réactifs hydrocarbonés, et de l'introduire directement dans le lit de catalyseur 5, pour éviter tout risque d'explosion. Il peut également être opportun de l'injecter de manière étagée à plusieurs niveaux dans le lit de catalyseur 5 pour un meilleur contrôle de la température et / ou de l'inflammabilité. Si un ou des composés faisant partie du flux réactionnel sont injectés sous forme liquide, il peut être approprié de les introduire directement dans le lit de catalyseur 5 (grâce aux moyens d'alimentation complémentaires 15) afin d'obtenir leur vaporisation immédiate au contact du solide.

Cela présente un avantage notamment pour les composés thermiquement instables (glycérol, acide lactique...), qui ont tendance à se dégrader à une température inférieure à leur température d'ébullition. En effet, dans ce cas le temps de trajet de ces composés à température élevée est minimisé, et ils sont mis à réagir simultanément à leur élévation de température et vaporisation (le transfert thermique au sein du lit de catalyseur étant très efficace du fait de la présence de particules solides).

En revanche, une injection sous la grille inférieure 14 est préférée pour les composés thermiquement stables.

Une injection directement dans le lit de catalyseur 5 est également appropriée pour les liquides qui peuvent cristalliser ou contenir des sels. Du fait des forces mécaniques d'attrition entre les grains de catalyseur, les particules solides qui se formeraient sont en effet ainsi détruites et éliminées du réacteur.

La zone de régénération 4 comprend une partie inférieure 6 et une partie supérieure 7, la partie supérieure 7 ayant généralement une section inférieure à la partie inférieure 6.

Dans la partie inférieure 6, le catalyseur est présent sous forme d'un lit fluidisé. La partie supérieure 7 peut être qualifiée d'élévateur, c'est-à-dire que le catalyseur y est entraîné (vers le haut) par transport pneumatique. En d'autres termes, la vitesse linéaire du gaz dans cette partie supérieure 7 est supérieure à la vitesse limite de chute des particules de catalyseur.

Un dispositif d'alimentation en flux de régénération 17 permet d'alimenter spécifiquement la zone de régénération 4 en flux de régénération, sur ou sous la grille inférieure 14.

Le flux de régénération peut être purement gazeux ou comprendre une composante liquide, et de préférence est purement gazeux. Il comprend une substance de régénération, c'est-à-dire apte à régénérer le catalyseur, qui est identique ou non à la substance de régénération éventuellement employée dans la zone de réaction 3, et qui de préférence est une substance oxydante telle que l'oxygène ; et il comprend éventuellement un ou plusieurs composés inertes.

Un flux de régénération contenant de l'oxygène est notamment approprié pour les catalyseurs qui se désactivent par cokage, car il permet la combustion du coke. De préférence, le flux de régénération comprend de l'oxygène et un ou des composés inertes caloporteurs (azote, vapeur d'eau, dioxyde de carbone...). Par exemple, le flux de régénération est de l'air.

Le débit du flux de régénération est adapté de telle sorte que le lit fluidisé dans la partie inférieure 6 de la zone de réaction 4 soit un lit fluidisé rapide ; la vitesse linéaire du gaz y est de préférence plus élevée que dans le lit de catalyseur 5 de la zone de réaction 3.

Le temps de séjour des particules de catalyseur dans la partie inférieure 6 de la zone de régénération 4 est largement supérieur au temps de séjour des particules de catalyseur dans la partie supérieure 7 de la zone de régénération 4 ; l'essentiel de la régénération a donc lieu dans la partie inférieure 6. La régénération du catalyseur dans la zone de régénération 4 n'est en général pas totale, elle est ajustée pour maintenir un degré moyen d'activation du catalyseur prédéterminé dans l'ensemble de la zone de réaction 3.

Les particules de catalyseur sont entraînés par transport pneumatique depuis l'extrémité supérieure de la partie inférieure 6 de la zone de régénération 4, de bas en haut le long de la partie supérieure 7 de la zone de régénération 4, et ce jusqu'à un premier dispositif de séparation gaz / solide 8.

Le premier dispositif de séparation gaz / solide 8 peut être un séparateur cyclonique. Il est connecté en sortie d'une part à un premier dispositif de soutirage de gaz 10, par lequel sont évacués les gaz d'échappement issus de la régénération ; et d'autre part à des premiers moyens de retour de catalyseur 9, par exemple une jambe de retour, assurant le retour du catalyseur dans la zone de réaction 3.

Dans la partie supérieure de la zone de réaction 3, un deuxième dispositif de séparation gaz / solide 11 est prévu, de préférence un séparateur cyclonique. Il est pourvu d'une bouche d'entrée 21 et est connecté en sortie d'une part à un deuxième dispositif de soutirage de gaz 13, par lequel est évacué un flux comprenant des produits de réaction ; et d'autre part à des deuxièmes moyens de retour des particules de catalyseur 12, par exemple une jambe de retour, assurant le retour du catalyseur dans la zone de réaction 3.

Une pluralité de tels dispositifs de séparation gaz / solide dans la zone de réaction 3 peuvent également être prévus.

Afin de permettre le passage du catalyseur de la zone de réaction 3 vers la zone de régénération 4, la paroi de séparation 2 ne vient pas au contact de la grille inférieure 14, mais au contraire un espacement est ménagé entre la partie inférieure de la paroi de séparation 2 et la grille inférieure 14.

Dans le mode de réalisation illustré, un chicanage 19 est prévu dans la zone de réaction 3, ménageant un espace intermédiaire 20 entre la paroi de séparation 2 et le chicanage 19. Lorsque la paroi de séparation 2 est tubulaire, le chicanage 19 entoure la paroi de séparation 2 (dans une portion inférieure de celle-ci) à distance de celle-ci. Le chicanage 19 peut avoir une forme tubulaire parallèle à la paroi de séparation 2, ou bien, comme cela est illustré, il peut être évasé vers le haut : ainsi la vitesse des particules de catalyseur dans l'espace intermédiaire 20 décroît lors de leur parcours vers le bas, et les bulles de gaz peuvent ainsi mieux s'échapper vers le reste de la zone de réaction 3, de sorte que l'entraînement de gaz vers la zone de régénération 4 est minimisé.

Des moyens d'injection de contre-pression 18 sont avantageusement prévus. Ils sont adaptés pour injecter un gaz dans l'espace intermédiaire 20. De la sorte, on peut réguler le flux de catalyseur de la zone de réaction 3 vers la zone de régénération 4 indépendamment du débit de flux de régénération dans la zone de régénération 4, et on contrecarre le passage du flux réactionnel dans la zone de régénération 4.

Le gaz injecté dans la zone intermédiaire 20 est de préférence un gaz inerte chimiquement. Il permet avantageusement de « stripper » le catalyseur (c'est-à-dire désorber en partie tous les réactifs adsorbés sur le catalyseur, mais surtout substituer le gaz riche en réactifs contenu dans et présent autour des particules de catalyseur), afin de limiter l'entraînement de réactifs vers la zone de régénération 4. Ce gaz peut être notamment de l'azote, du CO₂, de la vapeur d'eau, un mélange de ces gaz, ou encore du gaz de recyclage, obtenu en aval du procédé, et contenant majoritairement des gaz épurés du produit principal de la réaction effectuée dans le réacteur. De préférence le gaz est riche en vapeur d'eau, en CO₂ ou en azote. De manière encore plus préférée le gaz est de la vapeur d'eau.

Un système d'échange thermique, non représenté, comprenant par exemple des épingles de refroidissement, peut être prévu dans et / ou autour de l'enceinte 1. Il est également possible de prévoir un refroidissement spécifiquement pour la zone de réaction 3 et / ou la zone de régénération 4 (étant entendu que la température de régénération est généralement plus élevée que la température de réaction).

Le rapport de la section maximale de lit de catalyseur 5 dans la zone de réaction 3 sur la section maximale de la zone de régénération 4 (c'est-à-dire la section de la partie inférieure 6 de celle-ci, dans l'exemple illustré où cette partie inférieure 6 est cylindrique) ou des zones de régénération (en cas de pluralité) est supérieur ou égal à 1 et inférieur à 100, de préférence il est de 5 à 50, et plus particulièrement de 10 à 20. Un tel dimensionnement minimise le volume de réacteur dédié à la régénération et permet d'obtenir une efficacité optimale.

Avantageusement, le réacteur catalytique selon l'invention est adapté pour la mise en oeuvre des réactions ci-dessous, et en particulier pour la mise en oeuvre de réactions à une température inférieure ou égale à 600 °C et de préférence inférieure à 500°C.

### Réactions pouvant être mise en oeuvre selon l'invention

L'invention est avantageusement mise en oeuvre pour la production d'acide acrylique d'origine renouvelable, en particulier la production d'acide acrylique à partir de glycérol, comportant une première étape de déshydratation du glycérol en acroléine suivie d'une étape d'oxydation en phase gaz de l'acroléine ainsi obtenue ; ou dans la production d'acide acrylique par déshydratation des acides 2-hydroxypropionique (acide lactique) ou 3-hydroxypropionique et de leurs esters.

L'invention permet notamment de mettre en oeuvre un procédé de déshydratation du glycérol en acroléine. Dans ce type de procédé, le glycérol est alimenté sous forme d'une solution aqueuse avec une concentration de 20 à 95 % en masse. Plus le glycérol est concentré et plus les catalyseurs ont tendance à former du coke, ce qui nécessite de régénérer le catalyseur régulièrement. Selon le procédé décrit dans la demande WO 2006/087083, la réaction est avantageusement mise en oeuvre en présence d'oxygène, l'ajout d'oxygène dans la réaction de déshydratation de glycérol permettant de prolonger la durée de vie du catalyseur et d'espacer les régénérations.

La réaction est typiquement effectuée à une température de 220 à 350°C et de préférence de 280 à 320°C ; et à une pression variant de la pression atmosphérique à quelques bars (par exemple 5).

Les catalyseurs utilisables pour cette réaction sont des catalyseurs acides, ayant notamment une acidité de Hammett inférieure à +2, tels que décrits par exemple dans les documents EP 1 848 681, WO 2009/12855, WO 2009/044081 ou WO 2010/046227 De nombreux catalyseurs acides peuvent convenir pour cette réaction. On citera les zircones phosphatées, les zircones tungstées, les zircones silicées, les oxydes de titane ou d'étain imprégnés de tungstate ou phosphotungstate ou silicotungstate, les alumines ou silices phosphatées, les hétéropolyacides ou sels d'hétéropolyacides, les phosphates de fer et les phosphates de fer comprenant un promoteur.

Dans le cadre de ce procédé, le catalyseur subit une désactivation notamment par cokage. Cette désactivation peut toutefois être retardée par l'injection d'oxygène *in situ* dans la zone de réaction 3. Le rapport molaire O₂ / glycérol en entrée de réacteur est de 0,1 à 1,5 (de préférence de 0,3 à 1,0) et la pression partielle en oxygène est inférieure à 7 %.

Le catalyseur est régénéré en zone de régénération 4 par une injection d'oxygène.

L'invention permet également de mettre en oeuvre un procédé de déshydratation de l'acide lactique ou de l'acide 3-hydroxypropionique (et de leurs esters correspondants) en acide acrylique.

L'acide lactique a un point d'ébullition voisin de 217°C et l'acide 3-Hydroxypropionique a un point d'ébullition de 279°C (valeur calculée). Les limites d'inflammabilité pour l'acide lactique dans l'air sont de 3,1 % (Limite inférieure) et de 18 % (limite supérieure). L'ester méthylique de l'acide lactique a un point d'ébullition de 145°C, pour les limites d'inflammabilité de 1,1 et 3,6 % (ce qui donne une plus grande flexibilité d'utilisation que pour l'acide). L'ester méthylique de l'acide 3-hydroxypropionique a un point d'ébullition de 179°C (180°C en valeur calculée), L'ester éthylique de l'acide lactique a un point d'ébullition de 154°C, et des limites d'inflammabilité de 1,6 et 10,6 %. L'ester éthylique de l'acide 3-hydroxypropionique a un point d'ébullition de 187,5°C.

Pour ces réactions, on utilise une configuration de réacteur sensiblement identique à celle de la déshydratation du glycérol. Les conditions de déshydratation sont une température de 220 à 400°C, et de préférence de 250 à 350°C, et une pression de 0,5 à 5 bar.

Les catalyseurs qui peuvent convenir à ces réactions sont des catalyseurs acides, ayant notamment une acidité de Hammett inférieure à +2. Les catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides contenant notamment au moins un élément choisi parmi le groupe comprenant W, Mo et V. Parmi les oxydes mixtes, on peut citer particulièrement ceux à base de fer et de phosphore et ceux à base de césium, phosphore et tungstène.

D'autres catalyseurs qui peuvent aussi convenir pour ces réactions sont obtenus à partir de phosphates et/ou de sulfates des métaux alcalins, alcalino-terreux et des terres rares, et leurs mélanges. Dans ce groupe on trouve ainsi les phosphates et oxyphosphates de lanthane, les phosphates de sodium, les phosphates de calcium, le sulfate de calcium, le sulfate de magnésium, et les hydrogénophosphates correspondants. Le phosphate d'aluminium, le phosphate de bore. Toutes les matières actives citées ci-dessus peuvent être imprégnées ou enrobées sur tout type de support tel que : alumine, oxyde de titane, oxyde de zirconium, silice, mais aussi les oxydes mixtes correspondants, et le carbure de silicium.

La pression partielle en acide lactique ou 3-hydroxypropionique est généralement de 1 à 10 % et de préférence de 2 à 6 %.

Dans le cadre de ces réactions également, le catalyseur subit une désactivation notamment par cokage. Cette désactivation peut être retardée en injectant de l'oxygène *in situ* dans la zone de réaction 3. Les conditions relatives à l'injection d'oxygène sont les mêmes que celles décrites ci-dessus en relation avec la déshydratation du glycérol.

La régénération est effectuée par injection d'oxygène, comme décrit ci-dessus en relation avec la déshydratation du glycérol.

L'invention permet également de mettre en oeuvre un procédé de déshydratation de l'acide 2-hydroxyisobutyrique ou de l'acide 3-hydroxyisobutyrique en acide méthacrylique.

Pour ce type de réaction, on utilise une configuration de réacteur sensiblement identique à celle de la déshydratation du glycérol. Les conditions de déshydratation sont une température de 200 à 400°C, et de préférence de 250 à 350°C, et une pression de 0,5 à 5 bar. Les catalyseurs qui peuvent convenir à cette réaction sont des catalyseurs acides, ayant notamment une acidité de Hammett inférieure à +2. Les catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides contenant notamment au moins un élément choisi parmi le groupe comprenant W, Mo et V. Parmi les oxydes mixtes, on peut citer particulièrement ceux à base de fer et de phosphore et ceux à base de césium, phosphore et tungstène.

Des catalyseurs qui peuvent aussi convenir pour cette réaction sont obtenus à partir de phosphates et/ou de sulfates des métaux alcalins, alcalino-terreux et des terres rares, et leurs mélanges. Dans ce groupe on trouve ainsi les phosphates et oxyphosphates de lanthane, les phosphates de sodium, les phosphates de calcium, le sulfate de calcium, le sulfate de magnésium, et les hydrogénophosphates correspondants. Le phosphate d'aluminium, le phosphate de bore. Toutes les matières actives citées ci-dessus peuvent être imprégnées ou enrobées sur tout type de support tel que : alumine, oxyde de titane, oxyde de zirconium, silice, mais aussi les oxydes mixtes correspondants, et le carbure de silicium.

La pression partielle en acide (2- ou 3-)hydroxyisobutyrique est généralement de 1 à 20 % et de préférence de 2 à 10 %.

Dans le cadre de ces réactions également, le catalyseur subit une désactivation notamment par cokage. Cette désactivation peut être retardée en injectant de l'oxygène dans la zone de réaction 3. Les conditions relatives à l'injection d'oxygène sont les mêmes que celles décrites ci-dessus en relation avec la déshydratation du glycérol.

La régénération est effectuée par injection d'oxygène, comme décrit ci-dessus en relation avec la déshydratation du glycérol.

L'invention permet également de mettre en oeuvre des oxydations sélectives telles que l'oxydation du méthanol en formaldéhyde ou en diméthoxyméthane ; l'oxydation de l'éthanol en acétaldéhyde ou en diethoxyéthane ; l'oxydation de l'orthoxylène ou du naphtalène en anhydride phtalique, l'oxydation du benzène, du butène, du butanol ou du butane en anhydride maléique.

Pour ces réactions d'oxydation, la désactivation du catalyseur par cokage de produit relativement lentement, mais il existe également un phénomène de désactivation par sublimation d'oxydes de métaux. L'utilisation d'un lit fluidisé selon l'invention permet de faire tourner le catalyseur pour homogénéiser l'environnement et limiter l'apparition de points chauds, ce qui est particulièrement avantageux par rapport à un lit fixe.

Dans le cas des réactions d'oxydation du méthanol en formaldéhyde ou diméthoxyméthane, et d'oxydation de l'éthanol en acétaldéhyde ou diéthoxyéthane, les catalyseurs qui peuvent convenir sont des oxydes mixtes, tels que les oxydes de molybdène et de fer, les oxydes de molybdène contenant des métaux choisis parmi le bismuth, le vanadium, le tungstène, le cuivre, le nickel, le cobalt. Les conditions opératoires sont une température comprise entre 200 et 350°C, de préférence entre 250 et 300°C ; une pression comprise entre 1 et 5 bar. La pression partielle en alcool peut varier dans une large gamme de 3 à 50 %, et de préférence de 5 à 40 %, suivant le type de produit recherché. Dans le cas où les aldéhydes sont les produits recherchés, la pression partielle en alcool est comprise entre 3 et 10 % et de préférence entre 5 et 9 %. Dans le cas où les acétals sont les produits recherchés, la pression partielle en alcool est comprise entre 10 et 50 % et de préférence entre 20 et 40 %.

Dans le cas des réactions d'oxydation de l'orthoxylène et du naphtalène en anhydride phtalique, les catalyseurs sélectionnés contiennent de préférence du vanadium, et de préférence de l'oxyde de vanadium supporté. Les conditions opératoires sont une pression de 1 à 5 bar, et des températures de réaction de 280 à 450°C.

Dans le cas des réactions d'oxydation du butane, des butènes, du butanol et du benzène en anhydride maléique, les catalyseurs qui conviennent contiennent du vanadium, sous forme d'oxyde de vanadium supporté ou sous forme de d'oxyde mixte vanadium-phosphore supporté. Les températures des réactions sont de 350 à 500°C, et les pressions de 1 à 5 bar.

Dans le cas des réactions d'oxydation du propylène en acroléine, de l'isobutène ou du tertiobutanol en méthacroléine, les catalyseurs qui conviennent sont constitués majoritairement de molybdène, et contiennent des éléments choisis parmi (mais pas exclusivement) les éléments suivants : nickel, fer, cobalt, tungstène, potassium, bismuth, antimoine, chrome. Les températures de réaction sont comprises entre 320 et 450°C. Les pressions totales sont comprises entre 1 et 5 bar. Les pressions partielles en composé hydrocarboné sont comprises entre 5 et 15 %, et le rapport O₂ / composé hydrocarboné en entrée de réacteur est compris entre 0,5 et 4, et de préférence entre 0,8 et 2, et encore plus de préférence entre 1 et 1,8, et encore plus préféré entre 1,2 et 1,6.

Dans le cas des réactions d'oxydation de l'acroléine en acide acrylique, et de la méthacroléine en acide méthacrylique, les catalyseurs qui conviennent sont constitués majoritairement de molybdène, et contiennent des éléments choisis parmi les éléments suivants (mais pas exclusivement) : vanadium, tungstène, cuivre, antimoine, niobium, strontium, phosphore, fer. Les températures de fonctionnement sont comprises entre 250 et 350°C, pour une pression totale de 1 à 5 bar. La pression partielle en aldéhyde est comprise entre 5 et 15 %, et le ratio O₂ / aldéhyde en entrée de réacteur est compris entre 0,3 et 1,2, et de préférence compris entre 0,5 et 1.

D'autres réactions d'oxydation pouvant être mises en oeuvre selon l'invention sont :
- La production d'acide acrylique à partir de propylène et d'oxygène, les coproduits étant l'acroléine, l'acide acétique, l'acide maléique, l'acide propionique, l'acétaldéhyde et l'acétone, par exemple à une température de 300 à 400°C et à une pression de 1 à 3 bar.
- La production d'oxyde d'éthylène à partir d'éthylène et d'oxygène, les coproduits étant l'acétaldéhyde et le formaldéhyde, par exemple à une température de 230 à 290°C et à une pression de 10 à 30 bar.
- La production de 1,2-dichloroéthane à partir d'éthylène, d'acide chlorhydrique et d'oxygène, les coproduits étant le monoxyde de carbone, le chloral et divers composés chlorés, par exemple à une température de 220 à 300°C et à une pression de 2 à 6 bar.
- La production d'acide téréphtalique à partir de p-xylène et d'oxygène, les coproduits étant l'anhydride maléique, l'acide o-toluique et l'acide benzoïque, par exemple à une température de 175 à 230°C et à une pression de 15 à 30 bar.

Le réacteur selon l'invention peut aussi convenir pour des réactions d'ammoxydation mettant en jeu des mélanges ammoniac-oxygène-inerte-composé hydrocarboné. Les composés hydrocarbonés qui peuvent être utilisés comprennent le propylène, l'isobutène, l'acroléine, la méthacroléine, mais aussi des composés aromatiques. Les réactions d'ammoxydation sont effectuées à une température de 50 à 100°C plus élevée que les températures d'oxydation correspondantes.

A titre d'exemple, on peut produire de l'acrylonitrile (en coproduisant de l'acétonitrile, de l'acide hydrocyanique et du monoxyde de carbone) à partir de propylène et / ou propane, d'oxygène et d'ammoniac, par exemple à une température de 400 à 500°C et à une pression de 1 à 4 bar.

## Revendications

1. Réacteur catalytique comprenant une enceinte (1) et des moyens de séparation (2) disposés dans l'enceinte (1), délimitant au moins une zone de réaction (3) et au moins une zone de régénération (4), la zone de réaction (3) étant alimentée par un dispositif d'alimentation en flux réactionnel (15, 16) et étant connectée en sortie à un premier dispositif de soutirage de gaz (13), la zone de régénération (4) étant alimentée par un dispositif d'alimentation en flux de régénération (17) et étant connectée en sortie à un deuxième dispositif de soutirage de gaz (10), le réacteur comportant un lit fluidisé de particules de catalyseur (5) dans la zone de réaction (3), les moyens de séparation (2) permettant le passage des particules de catalyseur de la zone de réaction (3) vers la zone de régénération (4), et le réacteur comportant un entraînement pneumatique de particules de catalyseur dans la zone de régénération (4) permettant leur retour vers la zone de réaction (3), ledit réacteur étant **caractérisé en ce que** le rapport de la section maximale de lit de catalyseur dans la zone de réaction (3) sur la section maximale de la (ou des) zone(s) de régénération (4) va de 1 à 100, de préférence de 5 à 50, et plus particulièrement de 10 à 20.

2. Réacteur catalytique selon la revendication 1, dans lequel les moyens de séparation (2) comportent une paroi tubulaire, la zone de réaction (3) étant située à l'extérieur de la paroi tubulaire et la zone de régénération (4) étant située à l'intérieur de la paroi tubulaire.

3. Réacteur catalytique selon l'une des revendications 1 ou 2, dans lequel la zone de régénération (4) comprend une partie supérieure (7) et une partie inférieure (6), la partie supérieure (7) présentant une section inférieure à celle de la partie inférieure (6), la partie inférieure (6) comportant un lit fluidisé de particules catalyseur, et la partie supérieure (7) entraînant les particules de catalyseur par transport pneumatique.

4. Réacteur catalytique selon l'une des revendications 1 à 3, comprenant une grille inférieure (14) dans l'enceinte (1) adaptée à retenir les particules de catalyseur au-dessus de celle-ci, dans lequel un espacement est ménagé entre les moyens de séparation (2) et la grille inférieure (14), permettant le passage des particules de catalyseur de la zone de réaction (3) vers la zone de régénération (4).

5. Réacteur catalytique selon l'une des revendications 1 à 4, comprenant un chicanage (19) dans la zone de réaction (3) ménageant un espace intermédiaire (20) entre le chicanage (19) et les moyens de séparation (2).

6. Réacteur catalytique selon la revendication 5, comprenant des moyens d'injection de gaz de contre-pression (18) dans l'espace intermédiaire (20).

7. Procédé de réaction chimique mis en oeuvre dans un réacteur catalytique selon l'une des revendications 1 à 6, comprenant de manière concomitante :
- l'alimentation par un flux réactionnel d'un lit fluidisé (5) de particules de catalyseur dans une zone de réaction (3) disposée dans une enceinte (1) d'un réacteur,
- le soutirage d'un flux comprenant des produits de réaction en sortie de la zone de réaction (3),
- le passage, à l'aide de moyens de séparation (2), de particules de catalyseur de la zone de réaction (3) vers une zone de régénération (4) disposée à l'intérieur de l'enceinte (1) du réacteur, le rapport de la section maximale du lit fluidisé dans la zone de réaction (3) sur la section maximale de la zone de régénération (4) allant de 1 à 100,
- l'alimentation de la zone de régénération (4) par un flux de régénération (17)
- la régénération des particules de catalyseur et l'entraînement pneumatique des particules de catalyseur dans la zone de régénération (4),
- le soutirage d'un gaz d'échappement en sortie de la zone de régénération (4), et
- le retour des particules de catalyseur entraînées vers la zone de réaction (3) ; ladite réaction chimique étant **caractérisée en ce que** la durée de désactivation du catalyseur, définie comme étant la durée de réaction au bout de laquelle le catalyseur a perdu 25 % de son efficacité, est de 1 à 20 fois supérieure à la durée de régénération, définie comme étant la durée de régénération au bout de laquelle un catalyseur ayant perdu 25 % de son efficacité initiale retrouve jusqu'à 100 % de ladite efficacité initiale.

8. Procédé selon la revendication 7, dans lequel le flux de particules de catalyseur de la zone de réaction (3) vers la zone de régénération (4) est régulé par l'injection d'un gaz de contre-pression.

9. Procédé selon l'une des revendications 7 ou 8, dans lequel le flux de régénération comprend de l'oxygène, et dans lequel de préférence le flux réactionnel comprend de l'oxygène.

10. Procédé selon l'une des revendications 7 à 9, qui est :
- un procédé de déshydratation du glycérol en acroléine ; ou
- un procédé de déshydratation de l'acide lactique en acide acrylique ; ou
- un procédé de déshydratation de l'acide 3-hydroxypropionique en acide acrylique.
- un procédé de déshydratation de l'acide 3-hydroxyisobutyrique en acide methacrylique ; ou
- un procédé de déshydratation de l'acide 2-hydroxyisobutyrique, en acide methacrylique ; ou
- un procédé d'oxydation sélective telle que l'oxydation du méthanol en formaldéhyde ou en diméthoxyméthane ; l'oxydation de l'éthanol en acétaldéhyde ou en diethoxyéthane ; l'oxydation de l'orthoxylène ou du naphtalène en anhydride phtalique, l'oxydation du benzène, du butène, du butanol ou du butane en anhydride maléique.
- un procédé d'oxydation du propylène en acroléine, de l'isobutène ou du tertiobutanol en méthacroléine ; ou
- un procédé d'oxydation de l'acroléine en acide acrylique, et de la méthacroléine en acide méthacrylique.

## Patentansprüche

1. Katalytischer Reaktor, welcher einen Behälter (1) und in dem Behälter (1) angeordnete Trennmittel (2), die wenigstens eine Reaktionszone (3) und wenigstens eine Regenerationszone (4) begrenzen, umfasst, wobei die Reaktionszone (3) von einer Vorrichtung zur Zuführung eines Reaktionsstroms (15, 16) gespeist wird und am Ausgang mit einer ersten Gasabzugsvorrichtung (13) verbunden ist, wobei die Regenerationszone (4) von einer Vorrichtung zur Zuführung eines Regenerationsstroms (17) gespeist wird und am Ausgang mit einer zweiten Gasabzugsvorrichtung (10) verbunden ist, wobei der Reaktor eine Wirbelschicht von Katalysatorpartikeln (5) in der Reaktionszone (3) aufweist, wobei die Trennmittel (2) den Durchgang der Katalysatorpartikel aus der Reaktionszone (3) zur Regenerationszone (4) hin ermöglichen, und wobei der Reaktor eine pneumatische Mitnahme von Katalysatorpartikeln in der Regenerationszone (4) aufweist, die ihre Rückführung zur Reaktionszone (3) ermöglicht, wobei der Reaktor **dadurch gekennzeichnet ist, dass** das Verhältnis des maximalen Querschnitts des Katalysatorbetts in der Reaktionszone (3) zum maximalen Querschnitt der Regenerationszone(n) (4) 1 bis 100, vorzugsweise 5 bis 50 und insbesondere 10 bis 20 beträgt.

2. Katalytischer Reaktor nach Anspruch 1, wobei die Trennmittel (2) eine rohrförmige Wand umfassen, wobei sich die Reaktionszone (3) außerhalb der rohrförmigen Wand befindet und wobei sich die Regenerationszone (4) innerhalb der rohrförmigen Wand befindet.

3. Katalytischer Reaktor nach einem der Ansprüche 1 oder 2, wobei die Regenerationszone (4) einen oberen Teil (7) und einen unteren Teil (6) umfasst, wobei der obere Teil (7) einen Querschnitt aufweist, der kleiner als derjenige des unteren Teils (6) ist, wobei der untere Teil (6) eine Wirbelschicht aus Katalysatorpartikeln umfasst, und wobei der obere Teil (7) die Katalysatorpartikel durch pneumatischen Transport mitnimmt.

4. Katalytischer Reaktor nach einem der Ansprüche 1 bis 3, welcher einen unteren Rost (14) in dem Behälter (1) umfasst, der dafür ausgelegt ist, die Katalysatorpartikel oberhalb desselben zurückzuhalten, wobei ein Zwischenraum zwischen den Trennmitteln (2) und dem unteren Rost (14) ausgebildet ist, welcher den Durchgang der Katalysatorpartikel aus der Reaktionszone (3) zur Regenerationszone (4) hin ermöglicht.

5. Katalytischer Reaktor nach einem der Ansprüche 1 bis 4, welcher ein Ablenkteil (19) in der Reaktionszone (3) umfasst, wodurch ein dazwischenliegender Raum (20) zwischen dem Ablenkteil (19) und den Trennmitteln (2) ausgebildet ist.

6. Katalytischer Reaktor 5, welcher Mittel zur Injektion von Gegendruckgas (18) in den dazwischenliegenden Raum (20) umfasst.

7. Chemisches Reaktionsverfahren, welches in einem katalytischen Reaktor nach einem der Ansprüche 1 bis 6 durchgeführt wird und gleichzeitig umfasst:
- die Zuführung, durch einen Reaktionsstrom, einer Wirbelschicht (5) von Katalysatorpartikeln in eine Reaktionszone (3), die in einem Behälter (1) eines Reaktors angeordnet ist,
- das Abziehen eines Stroms, der Reaktionsprodukte umfasst, am Ausgang der Reaktionszone (3),
- den Durchgang, mithilfe von Trennmitteln (2), von Katalysatorpartikeln aus der Reaktionszone (3) zu einer Regenerationszone (4) hin, die im Inneren des Behälters (1) des Reaktors angeordnet ist, wobei das Verhältnis des maximalen Querschnitts der Wirbelschicht in der Reaktionszone (3) zum maximalen Querschnitt der Regenerationszone (4) 1 bis 100 beträgt,
- die Zuführung eines Regenerationsstroms (17) zu der Regenerationszone (4),
- die Regeneration der Katalysatorpartikel und die pneumatische Mitnahme der Katalysatorpartikel in der Regenerationszone (4),
- das Abziehen eines Abgases am Ausgang der Regenerationszone (4), und
- die Rückführung der mitgenommenen Katalysatorpartikel zur Reaktionszone (3);
wobei die chemische Reaktion **dadurch gekennzeichnet ist, dass** die Deaktivierungsdauer des Katalysators, die als die Reaktionsdauer definiert ist, an deren Ende der Katalysator 25 % seines Wirkungsgrades verloren hat, 1 bis 20 mal größer als die Regenerationsdauer ist, die als die Dauer der Regeneration definiert ist, an deren Ende ein Katalysator, nachdem er 25 % seines ursprünglichen Wirkungsgrades verloren hatte, 100 % dieses ursprünglichen Wirkungsgrades zurückgewonnen hat.

8. Verfahren nach Anspruch 7, wobei der Strom von Katalysatorpartikeln aus der Reaktionszone (3) zu der Regenerationszone (4) hin durch die Injektion eines Gegendruckgases geregelt wird.

9. Verfahren nach einem der Ansprüche 7 oder 8, wobei der Regenerationsstrom Sauerstoff umfasst und wobei vorzugsweise der Reaktionsstrom Sauerstoff umfasst.

10. Verfahren nach einem der Ansprüche 7 bis 9, welches:
- ein Verfahren zur Dehydratisierung von Glyzerin zu Acrolein ist; oder
- ein Verfahren zur Dehydratisierung von Milchsäure zu Acrylsäure ist; oder
- ein Verfahren zur Dehydratisierung von 3-Hydroxypropionsäure zu Acrylsäure ist; oder
- ein Verfahren zur Dehydratisierung von 3-Hydroxyisobuttersäure zu Methacrylsäure ist; oder
- ein Verfahren zur Dehydratisierung von 2-Hydroxyisobuttersäure zu Methacrylsäure ist; oder
- ein Verfahren zur selektiven Oxidation ist, wie die Oxidation von Methanol zu Formaldehyd oder zu Dimethoxymethan; die Oxidation von Ethanol zu Acetaldehyd oder zu Diethoxyethan; die Oxidation von ortho-Xylol oder von Naphthalin zu Phthalsäureanhydrid; die Oxidation von Benzol, von Buten, von Butanol oder von Butan zu Maleinsäureanhydrid,
- ein Verfahren zur Oxidation von Propen zu Acrolein, von Isobuten oder von tert-Butanol zu Methacrolein ist; oder
- ein Verfahren zur Oxidation von Acrolein zu Acrylsäure und von Methacrolein zu Methacrylsäure ist.

## Claims

1. A catalytic reactor comprising a chamber (1) and separating means (2) disposed in the chamber (1), delimiting at least one reaction zone (3) and at least one regeneration zone (4), the reaction zone (3) being supplied by a reaction stream feed device (15, 16) and being outlet-connected to a first gas withdrawal device (13), the regeneration zone (4) being fed by a regeneration stream feed device (17) and being outlet-connected to a second gas withdrawal device (10), the reactor comprising a fluidized bed of catalyst particles (5) in the reaction zone (3), the separating means (2) allowing the passage of the catalyst particles from the reaction zone (3) to the regeneration zone (4), and the reactor including an entrainment of catalyst particles into the regeneration zone (4) that allows them to return to the reaction zone (3), said reactor being **characterized in that** the ratio of the maximum cross section of catalyst bed in the reaction zone (3) to the maximum cross section of the regeneration zone (or zones) (4) is from 1 to 100, preferably from 5 to 50, and more particularly from 10 to 20.

2. The catalytic reactor as claimed in claim 1, wherein the separating means (2) comprise a tubular wall, the reaction zone (3) being situated outside the tubular wall and the regeneration zone (4) being situated inside the tubular wall.

3. The catalytic reactor as claimed in either of claims 1 and 2, wherein the regeneration zone (4) comprises a top part (7) and a bottom part (6), the top part (7) having a cross section lower than that of the bottom part (6), the bottom part (6) including a fluidized bed of catalyst particles, and the top part (7) entraining the catalyst particles.

4. The catalytic reactor as claimed in any of claims 1 to 3, comprising a lower grid (14) in the chamber (1) that is suitable for retaining the catalyst particles above said grid, wherein a clearance is made between the separating means (2) and the lower grid (14), allowing the passage of the catalyst particles from the reaction zone (3) to the regeneration zone (4).

5. The catalytic reactor as claimed in any of claims 1 to 4, comprising a baffle system (19) in the reaction zone (3) making a space (20) between the baffle system (19) and the separating means (2).

6. The catalytic reactor as claimed in claim 5, comprising means for injecting backpressure gas (18) into the space (20).

7. A chemical reaction process implemented in a catalytic reactor as claimed in any of claims 1 to 6, comprising concomitantly:
- feeding by a reaction stream of a fluidized bed (5) of catalyst particles into a reaction zone (3) disposed in a chamber (1) of a reactor,
- withdrawing a stream comprising reaction products at the outlet of the reaction zone (3),
- passing, using separating means (2), catalyst particles from the reaction zone (3) to a regeneration zone (4) disposed inside the chamber (1) of the reactor, the ratio of the maximum cross section of fluidized bed in the reaction zone (3) to the maximum cross section of the regeneration zone (4) is from 1 to 100,
- feeding the regeneration zone (4) by a regeneration stream (17),
- regenerating the catalyst particles and pneumatically entraining the catalyst particles into the regeneration zone (4),
- withdrawing an exhaust gas at the outlet of the regeneration zone (4), and
- returning the entrained catalyst particles to the reaction zone (3);
said chemical reaction being **characterized in that** the deactivation time of the catalyst, defined as being the reaction time after which the catalyst has lost 25% of its efficiency, is from 1 to 20 times greater than the regeneration time, defined as being the regeneration time after which a catalyst that has lost 25% of its initial efficiency regains up to about 100% of said initial efficiency.

8. The process as claimed in claim 7, wherein the stream of catalyst particles from the reaction zone (3) to the regeneration zone (4) is regulated by the injection of a backpressure gas.

9. The process as claimed in either of claims 7 and 8, wherein the regeneration stream comprises oxygen, and wherein the reaction stream preferably comprises oxygen.

10. The process as claimed in any of claims 7 to 9, which is:
- a process for dehydrating glycerol to acrolein; or
- a process for dehydrating lactic acid to acrylic acid; or
- a process for dehydrating 3-hydroxypropionic acid to acrylic acid;
- a process for dehydrating 3-hydroxyisobutyric acid to methacrylic acid; or
- a process for dehydrating 2-hydroxyisobutyric acid to methacrylic acid; or
- a process for selective oxidation such as the oxidation of methanol to formaldehyde or dimethoxymethane; the oxidation of ethanol to acetaldehyde or to diethoxyethane; the oxidation of ortho-xylene or naphthalene to phthalic anhydride, or the oxidation of benzene, butene, butanol, or butane to maleic anhydride;
- a process for oxidizing propylene to acrolein, and isobutene or tert-butanol to methacrolein; or
- a process for oxidizing acrolein to acrylic acid, and methacrolein to methacrylic acid.
